(19) Europäisches Patentamt
European Patent Office
Office européen des brevets

(11) Veröffentlichungsnummer: **0 293 716**
**A2**

# (12) EUROPÄISCHE PATENTANMELDUNG

(21) Anmeldenummer: **88108236.6**

(22) Anmeldetag: **24.05.88**

(51) Int. Cl.4: **C07D 209/90 , A61K 31/40**

(30) Priorität: **05.06.87 DE 3718892**

(43) Veröffentlichungstag der Anmeldung:
**07.12.88 Patentblatt 88/49**

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Rosentreter, Ulrich, Dr.**
**Kondorweg 23**
**D-5600 Wuppertal 1(DE)**
Erfinder: **Böshagen, Horst, Dr.**
**Wiesenstrasse 4**
**D-5657 Haan(DE)**
Erfinder: **Lieb, Folker, Dr.**
**Alfred-Kubin-Strasse 1**
**D-5090 Leverkusen(DE)**
Erfinder: **Oediger, Hermann, Dr.**
**Roggendorfstrasse 51**
**D-5000 Köln 80(DE)**
Erfinder: **Niewöhner, Ulrich, Dr.**
**Gartenstrasse 3**
**D-5632 Wermelskirchen(DE)**
Erfinder: **Seuter, Friedel, Dr.**
**Moospfad 16**
**D-5600 Wuppertal 1(DE)**
Erfinder: **Perzborn, Elisabeth, Dr.**
**Am Tescher Busch 13**
**D-5600 Wuppertal 11(DE)**
Erfinder: **Fiedler, Volker-Bernd, Dr.**
**Lehner Mühle 46**
**D-5090 Leverkkusen 3(DE)**

## (54) Polyhydrobenz(c,d)indolsulfonamide.

(57) Die neuen Polyhydrobenz[c,d]indol-sulfonamide können durch Umsetzung von N-substituierten Hexahydrobenzindol-sulfonamiden mit Acrylnitril und nachfolgender Oxidation oder Verseifung hergestellt werden. Die neuen Verbindungen können als Wirkstoffe in Arzneimitteln verwendet werden.

EP 0 293 716 A2

## Polyhydrobenz[c,d]indol-sulfonamide

Die Erfindung betrifft neue Polyhydrobenz[c,d]indol-sulfonamide, Verfahren zu ihrer Herstellung und ihre Verwendung in Arzneimitteln.

Es wurden neue Polyhydrobenz[c,d]indol-sulfonamide der allgemeinen Formel (I)

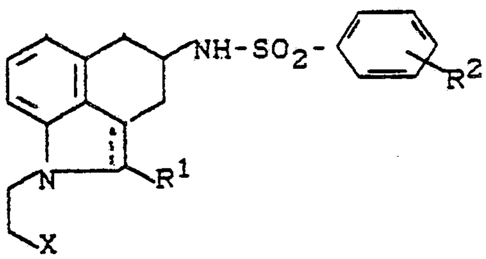

(I)

in welcher

$R^1$ - für Wasserstoff, Aryl oder Alkyl steht,

$R^2$ - für Wasserstoff, Halogen, Alkyl, Alkoxy, Trifluormethyl, Trifluormethoxy, Hydroxy, Aralkoxy oder

- für eine Gruppe der Formel

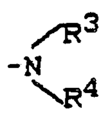

steht,

worin

$R^3$, $R^4$ - gleich oder verschieden sind und Wasserstoff, Alkyl, Aryl, Aralkyl oder Acyl bedeuten

und

X = für Cyano oder Carboxyl steht,

und deren Salze gefunden.

Die erfindungsgemäßen Stoffe zeigen überraschenderweise eine thrombozytenaggregationshemmende Wirkung und können zur therapeutischen Behandlung von Menschen und Tieren verwendet werden.

Die erfindungsgemäßen Polyhydrobenz[c,d]indol-sulfonamide können auch in Form ihrer Salze vorliegen. Im allgemeinen seien hier Salze mit anorganischen oder organischen Basen genannt.

Im Rahmen der vorliegenden Erfindung werden physiologisch unbedenkliche Salze bevorzugt. Physiologisch unbedenkliche Salze der Polyhydrobenz[c,d]indol-sulfonamide können Metall- oder Ammoniumsalze der erfindungsgemäßen Stoffe sein, welche eine freie Carboxylgruppe tragen (X = COOH). Besonders bevorzugt sind z.B. Natrium-, Kalium-, Magnesium- oder Calciumsalze, sowie Ammoniumsalze, die abgeleitet sind von Ammoniak oder organischen Amiden, wie beispielsweise Ethylamin, Diethylamin, Triethylamin, Diethanolamin, Triethanolamin, Dicyclohexylamin, Dimethylaminoethanol, Arginin oder Ethylendiamin.

Die erfindungsgemäßen Polyhydrobenz[c,d]indol-sulfonamide haben mehrere asymmetrische Kohlenstoffatome und können daher in verschiedenen stereoisomeren Formen existieren. Sowohl die einzelnen Isomeren als auch deren Mischungen sind Gegenstand der Erfindung. Beispielsweise seien die folgenden isomeren Formen der Polyhydrobenz[c,d]indol-sulfonamide genannt:

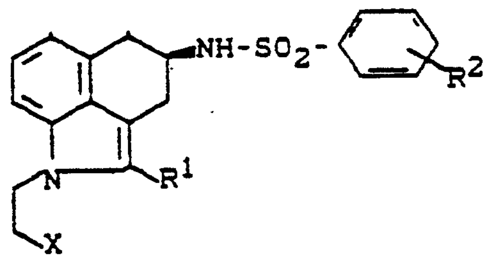

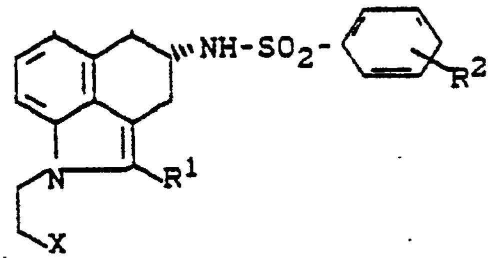

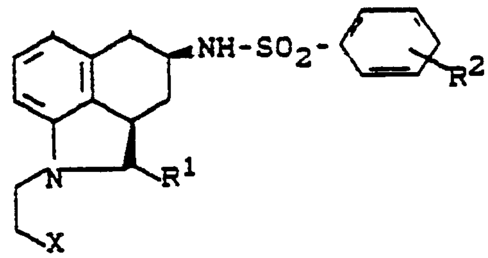

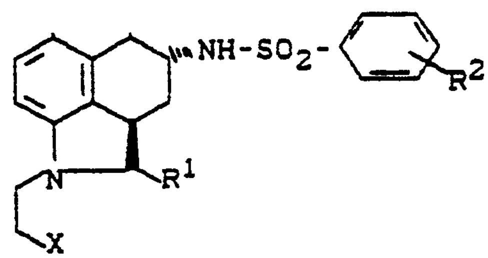

in welcher
$R^1$, $R^2$ und X die angegebene Bedeutung haben.

Alkyl steht im allgemeinen für einen verzweigten Kohlenwasserstoffrest mit 1 bis 12 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Aryl steht im allgemeinen für einen aromatischen Rest mit 6 bis etwa 12 Kohlenstoffatomen. Bevorzugte Arylreste sind Phenyl, Naphthyl und Biphenyl.

Aralkyl steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niederalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt:
Benzyloxy, Naphthylmethoxy, Phenethoxy und Phenylpropoxy.

Acyl steht im allgemeinen für Phenyl oder geradkettiges oder verzweigtes Niedrigalkyl mit 1 bis etwa 6

Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl.

Halogen steht im allgemeinen für Fluor, Chlor, Brom oder Iod, bevorzugt für Fluor, Chlor oder Brom. Besonders bevorzugt steht Halogen für Fluor oder Chlor.

Bevorzugt werden Verbindungen der allgemeinen Formel (I),
in welcher
$R^1$ - für Wasserstoff, Phenyl oder Niederalkyl steht,
$R^2$ - für Wasserstoff, Fluor, Chlor, Brom, Niederalkyl, Niederalkoxy, Trifluormethyl, Hydroxy, Benzyloxy oder
- für eine Gruppe der Formel

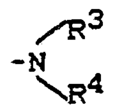

steht,
worin
$R^3$,$R^4$ - gleich oder verschieden sind und Wasserstoff, Niederalkyl, Phenyl, Benzyl oder Acetyl bedeuten
und
X - für Cyano oder Carboxyl steht,
und deren Salze.

Besonders bevorzugt sind solche Verbindungen der allgemeinen Formel (I),
in welcher
$R^1$ - für Wasserstoff, Methyl, Ethyl, Propyl oder Isopropyl steht,
$R^2$ - für Wasserstoff, Fluor, Chlor, Methyl, Ethyl, Propyl, Isopropyl, Methoxy, Ethoxy, Propoxy, Isopropoxy, Trifluormethyl oder
- für eine Gruppe der Formel

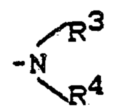

steht,
worin
$R^3$, $R^4$ - gleich oder verschieden sind und Wasserstoff, Methyl, Ethyl oder Propyl bedeuten
und
X - für Cyano oder Carboxyl steht,
und deren Salze.

Beispielhaft seien folgende Polyhydrobenz[c,d]indol-sulfonamide genannt:
1-(2-Cyanoethyl)-4-(4-fluorphenyl-sulfonamido)-1,2,2a,3,4,5-hexahydrobenz[c,d]indol
1-(2-Carboxyethyl)-4-(4-fluorphenyl-sulfonamido)-1,2,2a,3,4,5-hexahydrobenz[c,d]indol
1-(2-Cyanoethyl)-4-(4-fluorphenyl-sulfonamido)-1,3,4,5-tetrahydrobenz[c,d]indol
1-(2-Carboxyethyl)-4-(4-fluorphenyl-sulfonamido)-1,3,4,5-tetrahydrobenz[c,d]indol
1-(2-Cyanoethyl)-4-(4-chlorphenyl-sulfonamido)-1,2,2a,3,4,5-hexahydrobenz[c,d]indol
1-(2-Carboxyethyl)-4-(4-chlorphenyl-sulfonamido)-1,2,2a,3,4,5-hexahydrobenz[c,d]indol
1-(2-Cyanoethyl)-4-(4-chlorphenyl-sulfonamido)-1,3,4,5-tetrahydrobenz[c,d]indol
1-(2-Carboxyethyl)-4-(4-chlorphenyl-sulfonamido)-1,3,4,5-tetrahydrobenz[c,d]indol
1-(2-Cyanoethyl)-4-(4-methylphenyl-sulfonamido)-1,2,2a,3,4,5-hexahydrobenz[c,d]indol
1-(2-Carboxyethyl)-4-(4-methylphenyl-sulfonamido)-1,2,2a,3,4,5-hexahydrobenz[c,d]indol
1-(2-Cyanoethyl)-4-(4-methylphenyl-sulfonamido)-1,3,4,5-tetrahydrobenz[c,d]indol
1-(2-Carboxyethyl)-4-(4-methylphenyl-sulfonamido)-1,3,4,5-tetrahydrobenz[c,d]indol
1-(2-Cyanoethyl)-4-(4-methoxyphenyl-sulfonamido)-1,2,2a,3,4,5-hexahydrobenz[c,d]indol
1-(2-Carboxyethyl)-4-(4-methoxyphenyl-sulfonamido)-1,2,2a,3,4,5-hexahydrobenz[c,d]indol
1-(2-Cyanoethyl)-4-(4-methoxyphenyl-sulfonamido)-1,3,4,5-hexahydrobenz[c,d]indol
1-(2-Carboxyethyl)-4-(4-methoxyphenyl-sulfonamido)-1,3,4,5-hexahydrobenz[c,d]indol

1-(2-Cyanoethyl)-4-(3-fluorphenyl-sulfonamido)-1,2,2a,3,4,5-hexahydrobenz[c,d]indol
1-(2-Carboxyethyl)-4-(3-fluorphenyl-sulfonamido)-1,2,2a,3,4,5-hexahydrobenz[c,d]indol
1-(2-Cyanoethyl)-4-(3-fluorphenyl-sulfonamido)-1,3,4,5-tetrahydrobenz[c,d]indol
1-(2-Carboxyethyl)-4-(3-fluorphenyl-sulfonamido)-1,3,4,5-tetrahydrobenz[c,d]indol
1-(2-Cyanoethyl)-4-(3-chlorphenyl-sulfonamido)-1,2,2a,3,4,5-hexahydrobenz[c,d]indol
1-(2-Carboxyethyl)-4-(3-chlorphenyl-sulfonamido)-1,2,2a,3,4,5-hexahydrobenz[c,d]indol
1-(2-Cyanoethyl)-4-(3-chlorphenyl-sulfonamido)-1,3,4,5-tetrahydrobenz[c,d]indol
1-(2-Carboxyethyl)-4-(3-chlorphenyl-sulfonamido)-1,3,4,5-tetrahydrobenz[c,d]indol
1-(2-Cyanoethyl)-4-(3-methylphenyl-sulfonamido)-1,2,2a,3,4,5-hexahydrobenz[c,d]indol
1-(2-Carboxyethyl)-4-(3-methylphenyl-sulfonamido)-1,2,2a,3,4,5-hexahydrobenz[c,d]indol
1-(2-Cyanoethyl)-4-(3-methylphenyl-sulfonamido)-1,3,4,5-tetrahydrobenz[c,d]indol
1-(2-Carboxyethyl)-4-(3-methylphenyl-sulfonamido)-1,3,4,5-tetrahydrobenz[c,d]indol
1-(2-Cyanoethyl)-4-(3-methoxyphenyl-sulfonamido)-1,2,2a,3,4,5-hexahydrobenz[c,d]indol
1-(2-Carboxyethyl)-4-(3-methoxyphenyl-sulfonamido)-1,2,2a,3,4,5-hexahydrobenz[c,d]indol
1-(2-Cyanoethyl)-4-(3-methoxyphenyl-sulfonamido)-1,3,4,5-tetrahydrobenz[c,d]indol
1-(2-Carboxyethyl)-4-(3-methoxyphenyl-sulfonamido)-1,3,4,5-tetrahydrobenz[c,d]indol

Es wurde weiterhin ein Verfahren zur Herstellung der erfindungsgemäßen Polyhydrobenz[c,d]indol-sulfonamide der allgemeinen Formel (I)

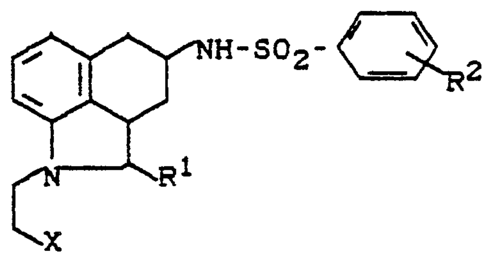

(I)

in welcher

$R^1$ - für Wasserstoff, Aryl oder Alkyl steht,

$R^2$ - für Wasserstoff, Halogen, Alkyl, Alkoxy, Trifluormethyl, Trifluormethoxy, Hydroxy, Aralkoxy oder - für eine Gruppe der Formel

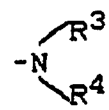

steht,

worin

$R^3$, $R^4$ - gleich oder verschieden sind und Wasserstoff, Alkyl, Aryl, Aralkyl oder Acyl bedeuten

und

X - für Cyano oder Carboxyl steht,

und deren Salze gefunden,

das dadurch gekennzeichnet ist, daß man

N-substituierte Hexahydrobenzoindol-sulfonamide der allgemeinen Formel (II)

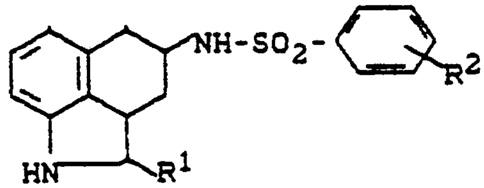

(II)

in welcher

$R^1$ und $R^2$ die oben angegebene Bedeutung haben,

mit Acrylnitril in Gegenwart eines inerten Lösemittels, gegebenenfalls in Anwesenheit eines Katalysators umsetzt,
dann im Fall der Herstellung der Tetrahydrobenzoindol-sulfonamide die Hexahydrobenzoindol-sulfonamide in Gegenwart eines inerten Lösemittels mit einem Oxidationsmittel umsetzt,
dann im Fall der Herstellung der Carboxyverbindungen (X = COOH) die Cyanoverbindungen (X = CN) verseift,
und dann im Fall der Herstellung der Salze mit den entsprechenden Basen umsetzt.

Das erfindungsgemäße Verfahren kann durch das folgende Formelschema erläutert werden:

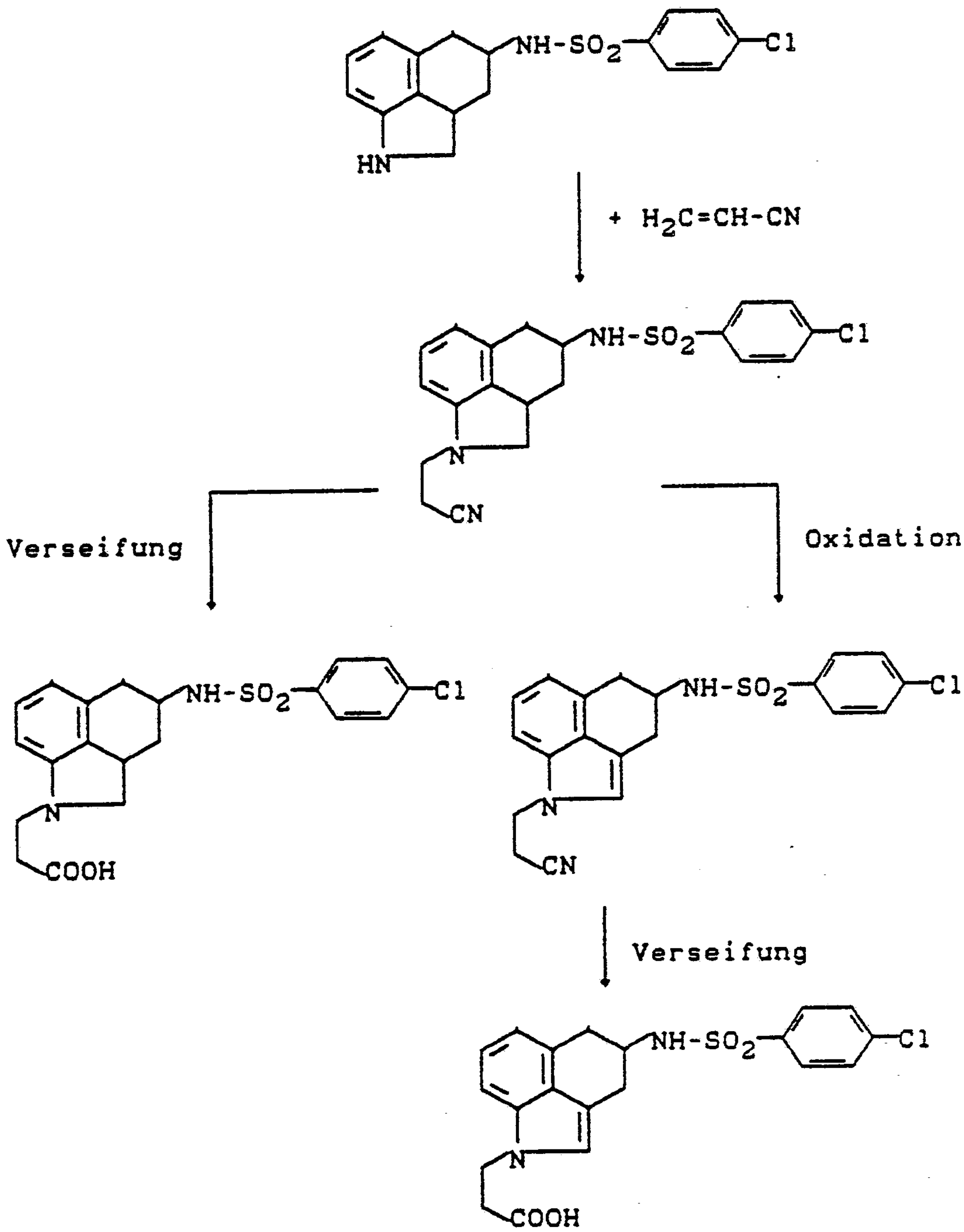


Die Tetrahydrobenzoindol-sulfonamide der Rahmen der Formel (I) entsprechen der Formel (Ia)

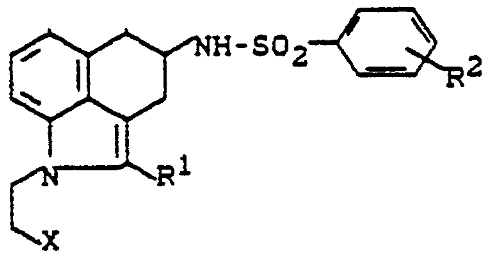

(Ia)

in der
$R^1$, $R^2$ und X die oben angegebene Bedeutung haben.

Die Hexahydrobenzoindol-sulfonamide im Rahmen der Formel (I) entsprechen der Formel (Ib)

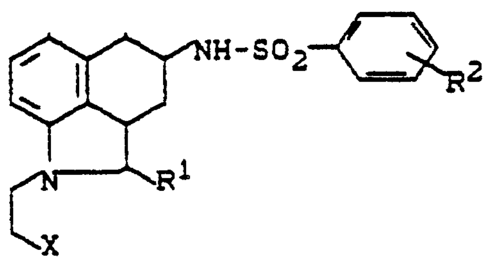

(Ib)

in der
$R^1$, $R^2$ und X die oben angegebene Bedeutung haben.

Bei der Durchführung des erfindungsgemäßen Verfahrens entstehen im allgemeinen Zwischenprodukte, die isoliert werden können. So ist es möglich, das erfindungsgemäße Verfahren in mehreren Verfahrensstufen auszuführen. Es kann aber auch möglich sein, verschiedene Verfahrensschritte zu kombinieren.

Geeignete Lösemittel für das Umsetzen mit Acrylnitril sind im allgemeinen die üblichen organischen Lösemittel, die sich unter den Reaktionsbedingungen nicht verändern. Hierzu gehören bevorzugt Alkohole wie Methanol, Ethanol, Propanol, Isopropanol oder Butanol, oder Kohlenwasserstoffe wie Benzol, Toluol, Xylol, Hexan, Cyclohexan oder Erdölfraktionen. Ebenso ist es möglich, Gemische der genannten Lösemittel zu verwenden. Es hat sich in einigen Fällen auch als günstig erwiesen, Acrylnitril in einem großen Überschuß direkt als Lösemittel einzusetzen.

Als Katalysatoren für die Umsetzung mit Acrylnitril eignen sich gegebenenfalls Kupfersalze, Quecksilbersalze, Palladiumsalze oder palladiumorganische Verbindungen. Hierzu gehören bevorzugt Kupferhalogenide wie beispielsweise Kupferchlorid, oder Kupfersulfat, Kupferacetat oder Kupferacetonylacetonat, oder Quecksilberhalogenide wie beispielsweise Quecksilberchlorid.

Die Umsetzung mit Acrylnitril wird im allgemeinen in einem Temperaturbereich von 0° C bis +200° C, bevorzugt von +50° C bis +150° C durchgeführt.

Die erfindungsgemäße Umsetzung mit Acrylnitril wird im allgemeinen bei Normaldruck durchgeführt. Es ist aber auch möglich, die Umsetzung bei Überdruck durchzuführen, z.B. bevorzugt in einem Bereich von 5000 bis 20000 bar, bevorzugt in einem Bereich von 7000 bis 15000 bar.

Im allgemeinen setzt man 1 bis 20 mol, bevorzugt 1 bis 10 mol Acrylnitril bezogen auf 1 mol N-unsubstituiertem Hexahydrobenzindol-sulfonamid ein. Besonders bevorzugt arbeitet man mit Acrylnitril als Lösemittel in einem bis zu 100-fachen, bevorzugt bis zu 50-fachen Überschuß.

Bei der Umsetzung der Tetrahydrobenzoindol-sulfonamide mit Oxidationsmitteln, eignen sich als Lösemittel die üblichen organischen Lösemittel, die sich unter den Reaktionsbedingungen nicht verändern. Hierzu gehören bevorzugt Kohlenwasserstoffe wie Benzol, Toluol, Xylol, Hexan, Cyclohexan oder Erdölfraktionen, oder Halogenkohlenwasserstoffe wie Methylenchlorid, Chloroform, Tetrachlorkohlenstoff, Dichlorethan oder Chlorbenzol. Ebenso ist es möglich, Gemische der genannten Lösemittel zu verwenden.

Als Oxidationsmittel eignen sich die üblichen anorganischen oder organischen Odxidationsmittel, die für die Überführung einer Hydroverbindung in eine Dehydroverbindung üblcherweise verwendet werden. Hierzu gehören bevorzugt anorganische Verbindungen wie Brom, Chlor oder Mangandioxid, oder organische Halogenverbindungen wie beispielsweise N-Chlorsuccinimid, N-Bromsuccinimid, Chloranil oder Dichlordicyanobenzochinon. Besonders bevorzugt verwendet man Chloranil.

Die Oxidation wird im allgemeinen in einem Temperaturbereich von +20° C bis +200° C, bevorzugt von +50° C bis +150° C durchgeführt.

Die Oxidation wird im allgemeinen bei Normaldruck durchgeführt. Es ist aber auch möglich, die Oxidation bei Unterdruck oder bei Überdruck durchzuführen (z.B. in einem Bereich von 0,5 bis 5 bar).

Im allgemeinen setzt man 1 bis 5, bevorzugt 2 bis 3 mol Oxidationsmittel bezogen auf 1 mol Hexahydrobenzoindolsulfonamid ein.

Die Verseifung der Cyanverbindung erfolgt in an sich bekannter Weise in Gegenwart von Basen, wie Alkali- oder Erdalkalihydroxide oder -alkoholaten, in inerten Lösemitteln wie Wasser oder Alkohol. Bevorzugt werden als Basen Natriumhydroxid, Kaliumhydroxid oder Bariumhydroxid, Natriummethanolat, Kaliummethanolat, Natriummethanolat oder Kaliumethanolat oder Kalium-tert.butanolat, bevorzugt in Wasser oder Methanol, Ethanol, Propanol oder Isopropanol, oder in Gemischen dieser Lösemittel verwendet.

Im allgemeinen setzt man 1 bis 100 mol, bevorzugt 2 bis 50 mol Base bezogen auf 1 mol der Cyanverbindung ein.

Die Verseifung wird im allgemeinen in einem Temperaturbereich von 0° C bis +100° C, bevorzugt von +20° C bis +80° C durchgeführt.

Als N-unsubstituierte Hexahydrobenzindol-sulfonamide der Formel (II) werden beispielsweise erfindungsgemäß eingesetzt:

4-(4-Fluorphenyl-sulfonamido)-1,2,2a,3,4,5-hexahydrobenz[c,d]indol
4-(4-Chlorphenyl-sulfonamido)-1,2,2a,3,4,5-hexahydrobenz[c,d]indol
4-(4-Methylphenyl-sulfonamido)-1,2,2a,3,4,5-hexahydrobenz[c,d]indol
4-(4-Methoxyphenyl-sulfonamido)-1,2,2a,3,4,5-hexahydrobenz[c,d]indol
4-(3-Fluorphenyl-sulfonamido)-1,2,2a,3,4,5-hexahydrobenz[c,d]indol
4-(3-Chlorphenyl-sulfonamido)-1,2,2a,3,4,5-hexahydrobenz[c,d]indol
4-(3-Methylphenyl-sulfonamido)-1,2,2a,3,4,5-hexahydrobenz[c,d]indol
4-(3-Methoxyphenyl-sulfonamido)-1,2,2a,3,4,5-hexahydrobenz[c,d]indol

Die N-unsubstituierten Hexahydrobenzindol-sulfonamide der allgemeinen Formel (II) sind neu.

Es wurde darüberhinaus ein Verfahren zur Herstellung der N-unsubstituierten Hexahydrobenzindol-sulfonamide der allgemeinen Formel (II)

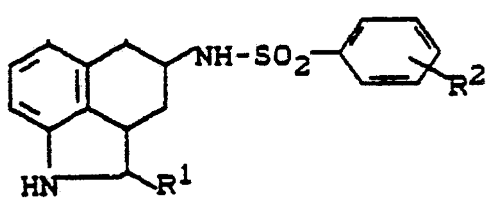

(II)

in welcher

$R^1$ - für Wasserstoff, Aryl oder Alkyl steht,

und

$R^2$ - für Wasserstoff, Halogen, Alkyl, Alkoxy, Trifluormethyl, Trifluormethoxy, Hydroxy, Aralkoxy oder
- für eine Gruppe der Formel

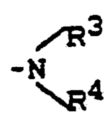

steht,

worin

$R^3$, $R^4$ - gleich oder verschieden sind und Wasserstoff, Alkyl, Aryl, Aralkyl oder Acyl bedeuten

gefunden,

das dadurch gekennzeichnet ist, daß man

Aminoverbindungen der allgemeinen Formel (III)

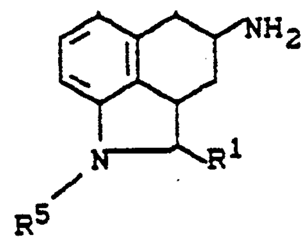

(III)

in welcher
$R^1$ die oben angegebene Bedeutung hat
und
$R^5$ - für Acyl, bevorzugt Benzoyl steht,
mit Sulfonsäurehalogeniden der allgemeinen Formel (IV)

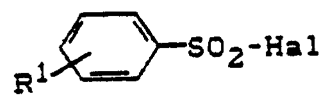

(IV)

in welcher
$R^1$ die oben angegebene Bedeutung hat
und
Hal - für Fluor, Chlor, Brom, Iod, bevorzugt für Chlor oder Brom steht,
in inerten Lösemitteln, gegebenenfalls in Anwesenheit von Basen umsetzt
und anschließend die Acylgruppe abspaltet.

Das erfindungsgemäße Verfahren kann durch folgendes Schema beispielhaft erläutert werden:

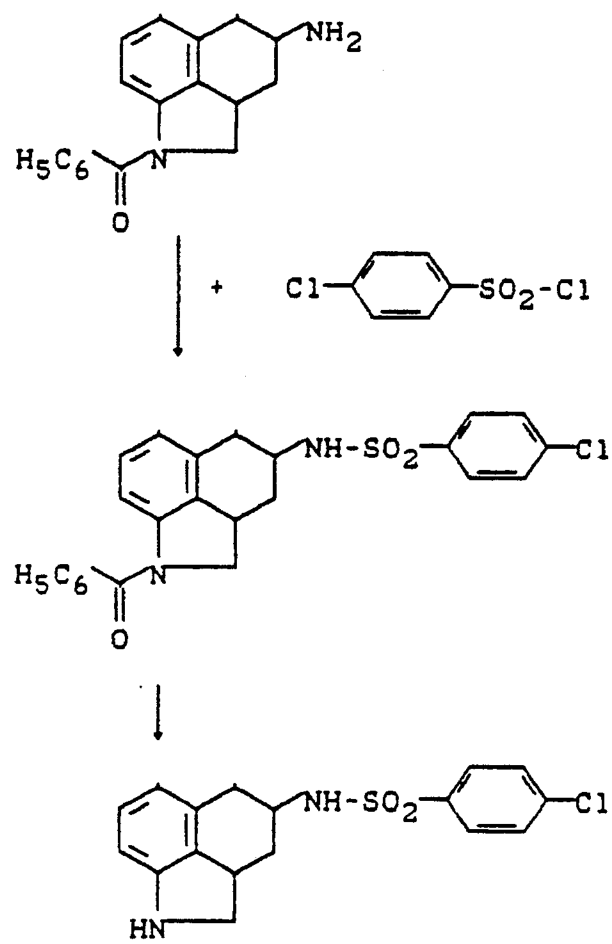

Als Lösemittel eignen sich hierbei die üblichen organischen Lösemittel, die sich unter den Reaktionsbedingungen nicht verändern. Hierzu gehören bevorzugt Ether wie Diethylether, Dioxan, Tetrahydrofuran oder Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, oder Amide wie Dimethylformamid, Dimethylacetamid, Hexamethylphosphorsäuretriamid, oder Acetonitril, Aceton oder Nitromethan. Ebenso ist es möglich, Gemische der genannten Lösemittel einzusetzen.

Als Basen können übliche basische Verbindungen eingesetzt werden. Hierzu gehören vorzugsweise Alkali- oder Erdalkalihydroxide wie Lithiumhydroxid, Natriumhydroxid, Kaliumhydroxid oder Bariumhydroxid, oder Alkalihydride wie Natriumhydrid, oder Alkali- oder Erdalkalicarbonate wie Natriumcarbonat, Natriumhydrogencarbonat, Kaliumcarbonat oder Calciumcarbonat, oder Alkalialkoholate wie beispielsweise Natriummethanolat, Natriumethanolat, Kaliummethanolat, Kaliumethanolat oder Kalium-tert.butanolat, oder Alkaliamide wie Lithiumdiisopropylamid oder Natriumamid, oder organische Amine wie Ethyldiisopropylamin, Benzyltrimethylammoniumhydroxid, Tetrabutylammoniumhydroxid, Pyridin, Dimethylaminopyridin, Triethylamin, N-Methylpiperidin, 1,5-Diazabicyclo[4,3,0]non-5-en oder 1,5-Diazabicyclo[5,4,0]undec-5-en.

Das erfindungsgemäße Verfahren wird im allgemeinen in einem Temperaturbereich von -30° C bis +150° C, bevorzugt von +20° C bis +80° C durchgeführt.

Das erfindungsgemäße Verfahren wird im allgemeinen bei Normaldruck durchgeführt. Es ist aber auch möglich, bei Unterdruck oder bei Überdruck zu arbeiten (z.B. in einem Bereich von 0,5 bis 200 bar).

Im allgemeinen setzt man 1 bis 5 mol, bevorzugt 1 bis 2 mol, besonders bevorzugt 1 mol Sulfonsäurehalogenid bezogen auf 1 mol der Aminoverbindung ein.

Die Acylgruppe kann beispielsweise durch Zusatz von Basen abgespalten werden. Als Basen eignen sich hierbei die üblichen anorganischen Basen wie Alkali- oder Erdalkalihydroxide beispielsweise Natriumhydroxid, Kaliumhydroxid, Bariumhydroxid, oder Alkalicarbonate wie Natriumcarbonat oder Kaliumcarbonat. Als Lösemittel eignen sich hierbei Wasser oder Alkohole wie Methanol, Ethanol, Propanol, Isopropanol oder Butanol. Ebenso können Gemische der genannten Lösemittel eingesetzt werden. Man setzt im allgemeinen 1 bis 5 mol, bevorzugt 2 bis 4 mol der Base bezogen auf 1 mol der Acylverbindung ein. Die Abspaltung der Acylgruppe erfolgt im allgemeinen in einem Temperaturbereich von 0° C bis +150° C, bevorzugt von +20° C bis +100° C.

Die als Ausgangsstoffe eingesetzten Aminoverbindungen der allgemeinen Formel (III) sind bekannt oder können nach bekannten Methoden hergestellt werden. Die Herstellung kann durch folgendes Reaktionsschema erläutert werden:

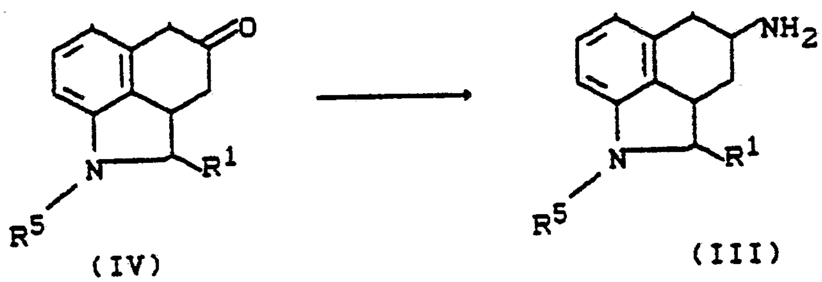

(IV) (III)

Danach werden Ketone der allgemeinen Formel (V) nach üblichen Methoden in inerten Lösemitteln, gegebenenfalls in Anwesenheit von Hilfsstoffen mit ammoniakhaltigen Komponenten reduktiv aminiert.

Als Lösemittel eignen sich hierbei die üblichen organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Tetrahydrofuran oder Dioxan, oder Chlorkohlenwasserstoffe wie Methylenchlorid oder Chloroform, oder Acetonitril, Dimethylformamid, Dimethylsulfoxid, Eisessig oder Gemische der genannten Lösemittel.

Als Reduktionsmittel eignen sich die üblichen komplexen Hydride wie beispielsweise Natriumborhydrid, Natruimcyanoborhydrid, oder Aminoborankomplexe, oder auch Wasserstoff, gegebenenfalls in Anwesenheit eines Metallkatalysators wie Raney-Nickel oder Palladium.

Als ammoniakhaltige Komponente werden wäßrige Ammoniaklösung, gasförmiger Ammoniak, oder auch Ammoniumsalze wie Ammoniumchlorid, Ammoniumsulfat, Ammoniumacetat oder Ammoniumformiat eingesetzt.

Die Umsetzung kann bei normalem, erhöhtem oder bei erniedrigtem Druck durchgeführt werden (0,5 bis 5 bar). Im allgemeinen arbeitet man bei der Verwendung von komplexen Metallhydriden bei normalem

Druck, bei der Verwendung von Wasserstoff mit Überdruck.

Die reduktive Aminierung wird im allgemeinen in einem Temperaturbereich von 0° C bis +150° C, bevorzugt von +20° C bis +100° C durchgeführt.

Die als Ausgangsstoffe eingesetzten Ketone der allgemeinen Formel (V) sind bekannt oder können nach bekannten Methoden hergestellt werden [ Kornfeld, Woodward et al., J. Amer. Chem. Soc. 78, 3096 (1956)].

Die neuen Polyhydrobenzo[c,d]indol-sulfonamide bzw. deren Salze können als Wirkstoffe in Arzneimitteln eingesetzt werden. Die Wirkstoffe weisen eine thrombozytenaggregationshemmende Wirkung auf. Sie können bevorzugt zur Behandlung von Thrombosen, Thromboembolien, Ischämien, als Antiasthmatika und als Antiallergika eingesetzt werden.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeignete. Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:
Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verarbreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Beispiel 1

A. 1-Beonzyl-4-amino-1,2,2a,3,4,5-hexahydrobenz[c,d]indol

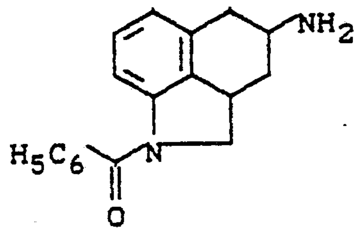

3,5 g 1-Benzoyl-4-oxo-1,2,2a,3,4,5-hexahydro[c,d]indol werden zusammen mit 9,7 g Ammoniumacetat in einer Mischung von 35 ml Dichlormethan und 35 ml Methanol 2 h gerührt. Dann werden 0,79 g Natriumcyanoborhydrid zugegeben und die Reaktionslösung 1 h bei Raumtemperatur gerührt. Zur Aufarbeitung wird mit 150 ml Essigester verdünnt und 4 mal mit 2n Schwefelsäure extrahiert. Die vereinigten wäßrigen Phasen werden mit Essigester gewaschen und mit 20%iger Natronlauge alkalisch gestellt. Es wird 4 mal mit Dichlormethan extrahiert, mit Natriumsulfat getrocknet und eingedampft. Der Rückstand wird an 20 g Kieselgel-60 mit Toluol/Ethanol als Eluens (schrittweise Änderung des Verhältnisses von 20:1 auf 10:1, dann auf 1:1) chromatographiert. Man erhält so eine Fraktion, die nach Eindampfen 2,41 g sauberes Produkt als festen Rückstand ergibt.
Ausbeute: 68,7% der Theorie
Fp.: 126° C
$R_f$-Wert: 0,15 (Toluol:Ethanol 1:1)

B. 1-Benzoyl-4-(4-fluorphenylsulfonamido)-1,2,2a,3,4,5 -hexahydrobenz[c,d]indol

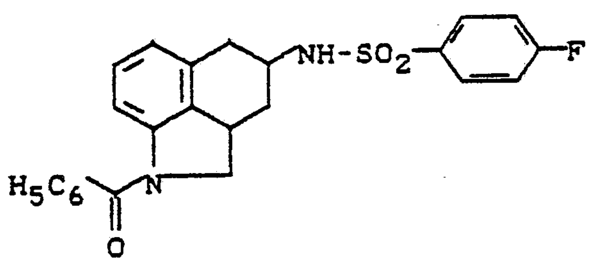

13,1 g 1-Benzoyl-4-amino-1,2,2a,3,4,5-hexahydrobenz[c,d]indol werden zusammen mit 6,06 g Triethylamin in 700 ml Dichlormethan gelöst. Dazu werden bei Raumtemperatur 10 g 4-Fluorbenzolsulfonsäurechlorid in 100 ml Dichlormethan zugetropft. Es wird 1 h bei Raumtemperatur nachgerührt. Dann wird 2 mal mit 2n Schwefelsäure, 1 mal mit Wasser, 2 mal mit 2 n Natronlauge und 1 mal mit Wasser gewaschen. Nach Trocknen mit Natriumsulfat und eindampfen erhält man einen Rückstand, der aus Essigester- Ether-Gemisch 15,3 g kristallines Produkt ergibt.
Ausbeute: 75,1% der Theorie
Fp.: 172° C
$R_f$-Wert: 0,39 (Toluol:Ethanol 6:1)

C. 4-(4-Fluorphenylsulfonamido)-1,2,2a,3,4,5-hexahydrobenz[c,d]indol

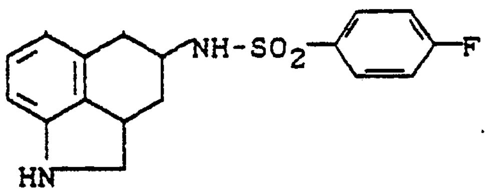

15 g 1-Benzoyl-4-(4-fluorphenylsulfonamido)-1,2,2a,3,4,5-hexahydrobenz[c,d]indol werden in einer Mischung von 350 ml 10%iger Kalilauge und 370 ml Isopropanol 24 h unter Rückfluß erhitzt. Nach Abkühlen wird die Reaktionslösung im Vakuum zur Hälfte eingedampft, mit 1 l Wasser versetzt und 4 mal mit Essigester extrahiert. Die vereingten organischen Phasen werden mit Natriumsulfat getrocknet und eingedampft. Der feste Rückstand wird aus Toluol auskristallisiert. Man erhält so 9,2 g kristallines Produkt.
Ausbeute: 80,7% der Theorie
Fp.: 168 °C
$R_f$-Wert: 0,27 (Toluol:Essigester 8:2)

D. 1-(2-Cyanoethyl)-4-(4-fluorphenylsulfonamido)-1,2,2a,3,4,5-hexahydrobenz[c,d]indol

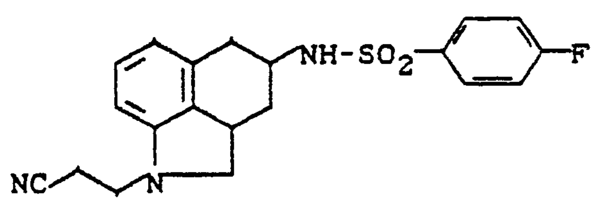

7,9 g 4-(4-Fluorphenylsulfonamido)-1,2,2a,3,4,5-hexahydrobenz[c,d]indol werden in 80 ml frisch destilliertem Acrylnitril gelöst und mit 0,7 g Kupfer(II)acetat versetzt. Die Reaktionslösung wird 1 h unter Rückfluß erhitzt. Nach Abkühlen wird im Vakuum eingeengt und der Rückstand über 20 g Kieselgel-60 mit Toluol-Essigester im Verhältnis 10:1 bis 6:1 als Eluens chromatographiert. Man erhält so eine Fraktion, die nach Eindampfen 7 g Produkt als öligen Rückstand ergibt.
Ausbeute: 76,4% der Theorie
$R_f$-Wert: 0,5 (Toluol:Ethanol 6:1)

Beispiel 2

1-(2-Carboxyethyl)-4-(4-fluorphenylsulfonamido)-1,2,2a,3,4,5-hexahydrobenz[c,d]indol

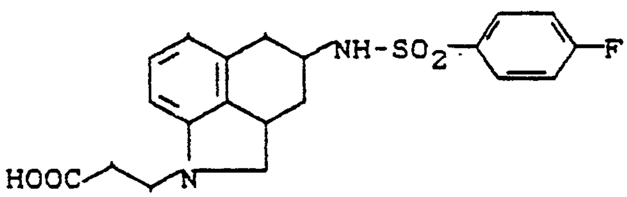

3,5 g 1-(2-Cyanoethyl)-4-(4-fluorphenylsulfonamido)-1,2,2a,3,4,5-hexahydrobenz[c,d]indol werden in einer Mischung von 70 ml Isopropanol mit 100 ml 10%iger Kalilauge 4 h unter Rückfluß erhitzt. Die

Reaktionslösung wird im Vakuum eingeengt, mit 100 ml Wasser verdünnt und 2 mal mit Essigester extrahiert. Die wäßrige Phase wird mit 6n Salzsäure sauer gestellt und 3 mal mit Essig ester extrahiert. Die vereinigten organischen Phasen werden mit Natriumsulfat getrocknet und eingedampft. Der Rückstand ergibt nach Umkristallisation aus Isopropanol 2,2 g kristallines Produkt.
Ausbeute: 60% der Theorie
Fp.: 137° C
$R_f$-Wert: 0,57 (Toluol:Ethanol 3:1)

Beispiel 3

1-(2-Cyanoethyl)-4-(4-fluorphenylsulfonamido)-1,3,4,5-tetrahydrobenz[c,d]indol

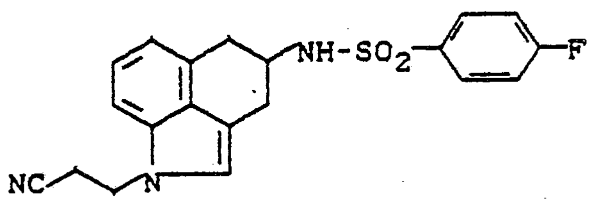

3,5 g 1-(2-Cyanoethyl)-4-(4-fluorphenylsulfonamido)-1,2,2a,3,4,5-hexyhydrobenz[c,d]indol werden zusammen mit 3 g Chloranil 24 h unter Rückfluß erhitzt. Nach Abkühlen wird 3 mal mit 1 n Natronlauge gewaschen, mit Natriumsulfat getrocknet und eingedampft. Der Rückstand wird an 30 g Kieselgel-60 mit Toluol-Essigester im Verhältnis 10:1 als Eluens chromatographiert. Man erhält so eine Fraktion, die nach Eindampfen 1,43 g öliges Produkt ergibt.
Ausbeute: 40% der Theorie
$R_f$-Wert: 0,44 (Toluol:Aceton 3:1)

Beispiel 4

1-(2-Carboxyethyl)-4-(4-fluorphenylsulfonamido)-1,3,4,5-tetrahydrobenz[c,d]indol

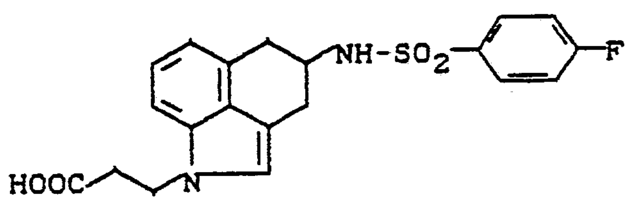

1,4 g 1-(2-Cyanoethyl)-4-(4-fluorphenylsulfonamido)-1,3,4,5-tetrahydrobenz[c,d]indol werden in einer Mischung von 30 ml Isopropanol und 40 ml 10%iger Kalilauge 4 h unter Rückfluß erhitzt. Die Reaktionslösung wird im Vakuum eingeengt, mit 100 ml Wasser verdünnt und 2 mal mit Essigester extrahiert. Die wäßrige Phase wird mit 6n Salzsäure sauer gestellt und 3 mal mit Essigester extrahiert. Nach Trocknen mit Natriumsulfat und Eindampfen wird der Rückstand zur Überführung in sein Natriumsalz in 20 ml Methanol gelöst, mit 3,1 ml in NaOH versetzt und im Vakuum zur Trockne eingedampft. Man erhält so 1,3 g festes Natriumsalz.
Ausbeute: 84% der Theorie
$R_f$-Wert: 0,27 (Toluol:Ethanol 6:1)

Beispiel 5

Zur Bestimmung der thrombozytenaggregationshemmenden Wirkung wurde Blut von gesunden Probanden beiderlei Geschlechts verwendet. Als Antikoagulans wurden einem Teil 3,8%iger wäßri ger Natriumzitratlösung 9 Teile Blut zugemischt. Mittels Zentrifugation erhält man aus diesem Blut plättchenreiches Zitratplasma (PRP) (Jürgens/Beller, Klinische Methoden der Blutgerinnungsanalyse; Thieme Verlag, Stuttgart, 1959).

Für diese Untersuchungen wurden 0,8 ml PRP und 0,1 ml der Wirkstofflösung bei 37 °C im Wasserbad vorinkubiert. Anschließend wurde die Thrombozytenaggregation nach der turbidometrischen Methode (Borth, B.V.R., J. Physiol. (London), 162, 67, 1962) im Aggregometer bei 37 °C bestimmt (Therapeutische Berichte 47, 80-86, 1975). Hierzu wurde die vorinkubierte Probe mit 0,1 ml Kollagen, einem aggregationsauslösenden Agens, versetzt. Die Veränderung der optischen Dichte in der Probe der PRP wurde während einer Zeitdauer von 6 Minuten aufgezeichnet und der Ausschlag nach 6 Minuten bestimmt. Hierzu wird die prozentuale Hemmung gegenüber der Kontrolle errechnet.
Als Grenzkonzentration wird der Bereich der minimal effektiven Konzentration angegeben (Tabelle 3).

Tabelle 3

| Beispiel-Nr. | Hemmung mg/l (Grenzkonzentration) |
|---|---|
| 2 | 0,03 - 0,1 |

**Ansprüche**

1. Polyhydrobenz[c,d]indol-sulfonamide der Formel

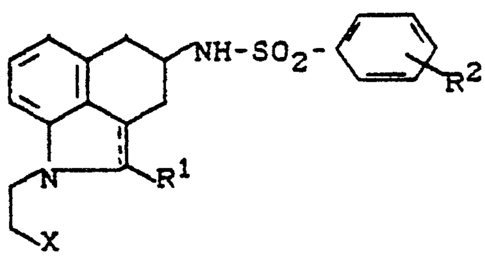

(I)

in welcher
$R^1$ - für Wasserstoff, Aryl oder Alkyl steht,
$R^2$ - für Wasserstoff, Halogen, Alkyl, Alkoxy, Trifluormethyl, Trifluormethoxy, Hydroxy, Aralkoxy oder
- für eine Gruppe der Formel

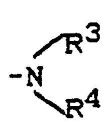

steht,
worin
$R^3$, $R^4$ - gleich oder verschieden sind und Wasserstoff, Alkyl, Aryl, Aralkyl oder Acyl bedeuten
und
X - für Cyano oder Carboxyl steht,
und deren Salze.

2. Polyhydrobenz[c,d]indol-sulfonamide nach Anspruch 1,
worin
$R^1$ - für Wasserstoff, Phenyl oder Niederalkyl steht,
$R^2$ - für Wasserstoff, Fluor, Chlor, Brom, Niederalkyl, Niederalkoxy, Trifluormethyl, Hydroxy, Benzyloxy oder
- für eine Gruppe der Formel

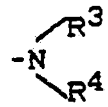

steht,
worin
$R^3$,$R^4$ - gleich oder verschieden sind und Wasserstoff, Niederalkyl, Phenyl, Benzyl oder Acetyl bedeuten
und
X - für Cyano oder Carboxyl steht,
und deren Salze.

3. Polyhydrobenz[c,d]indol-sulfonamide nach den Ansprüchen 1 und 2,
worin
$R^1$ - für Wasserstoff, Methyl, Ethyl, Propyl oder Isopropyl steht,
$R^2$ - für Wasserstoff, Fluor, Chlor, Methyl, Ethyl, Propyl, Isopropyl, Methoxy, Ethoxy, Propoxy, Isopropoxy, Trifluormethyl oder
- für eine Gruppe der Formel

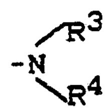

steht,
worin
$R^3$, $R^4$ - gleich oder verschieden sind und Wasserstoff, Methyl, Ethyl oder Propyl bedeuten
und
X - für Cyano oder Carboxyl steht,
und deren Salze.

4. Polyhydrobenz[c,d]indol-sulfonamide der Formel

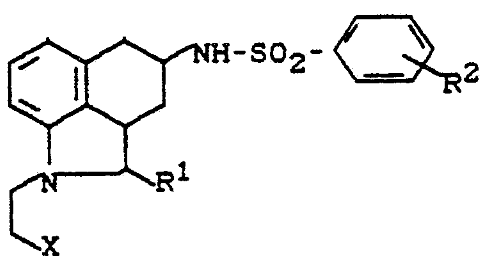

(I)

in welcher
$R^1$ - für Wasserstoff, Aryl oder Alkyl steht,
$R^2$ - für Wasserstoff, Halogen, Alkyl, Alkoxy, Trifluormethyl, Trifluormethoxy, Hydroxy, Aralkoxy oder
- für eine Gruppe der Formel

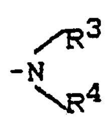

steht,
worin
$R^3$, $R^4$ - gleich oder verschieden sind und Wasserstoff, Alkyl, Aryl, Aralkyl oder Acyl bedeuten
und
X - für Cyano oder Carboxyl steht,
und deren Salze,
zur therapeutischen Behandlung.

5. Verfahren zur Herstellug von Polyhydrobenz[c,d]indol-sulfonamide der Formel

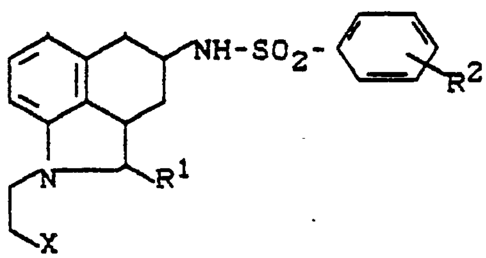

(I)

in welcher
$R^1$ - für Wasserstoff, Aryl oder Alkyl steht,
$R^2$ - für Wasserstoff, Halogen, Alkyl, Alkoxy, Trifluormethyl, Trifluormethoxy, Hydroxy, Aralkoxy oder
- für eine Gruppe der Formel

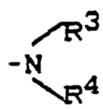

steht,
worin
$R^3$, $R^4$ - gleich oder verschieden sind und Wasserstoff, Alkyl, Aryl, Aralkyl oder Acyl bedeuten
und
X - für Cyano oder Carboxyl steht,
und deren Salze,
dadurch gekennnzeichnet, daß man N-substituierte Hexahydrobenzoindol-sulfonamide der allgemeinen Formel (II)

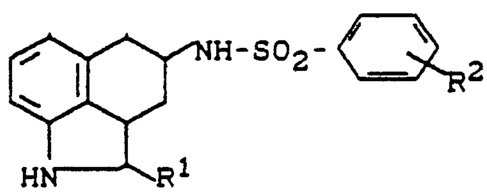

(II)

in welcher
$R^1$ und $R^2$ die oben angegebene Bedeutung haben,
mit Acrylnitril in Gegenwart eines inerten Lösemittels, gegebenenfalls in Anwesenheit eines Katalysators umsetzt,
dann im Fall der Herstellung der Tetrahydrobenzoindol-sulfonamide die Hexahydrobenzoindol-sulfonamide in Gegenwart eines inerten Lösemittels mit einem Oxidationsmittel umsetzt,
dann im Fall der Herstellung der Carboxyverbindungen (X = COOH) die Cyanoverbindungen (X = CN) verseift,
und dann im Fall der Herstellung der Salze mit den entsprechenden Basen umsetzt.

6. Verfahren nach Anspruch 5, dadurch gekennzeichnet, daß man die Umsetzung mit Acrylnitril im Temperaturbereich von 0° C bis 200° C durchführt.

7. Hexahydrobenzindol-sulfonamide der Formel

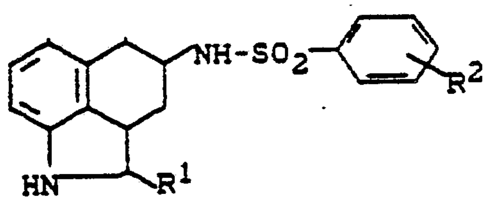

(II)

in welcher
$R^1$ - für Wasserstoff, Aryl oder Alkyl steht,
und
$R^2$ - für Wasserstoff, Halogen, Alkyl, Alkoxy, Trifluormethyl, Trifluormethoxy, Hydroxy, Aralkoxy oder
- für eine Gruppe der Formel

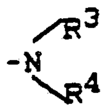

steht,
worin
$R^3$, $R^4$ - gleich oder verschieden sind und Wasserstoff, Alkyl, Aryl, Aralkyl oder Acyl bedeuten.

8. Verfahren zur Herstellung von N-unsubstituierten Hexahydrobenzindol-sulfonamiden der Formel

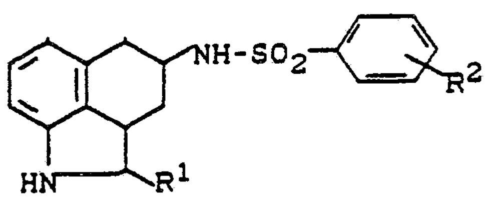

(II)

in welcher
$R^1$ - für Wasserstoff, Aryl oder Alkyl steht,
und
$R^2$ - für Wasserstoff, Halogen, Alkyl, Alkoxy, Trifluormethyl, Trifluormethoxy, Hydroxy, Aralkoxy oder
- für eine Gruppe der Formel

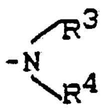

steht,
worin
$R^3$, $R^4$ - gleich oder verschieden sind und Wasserstoff, Alkyl, Aryl, Aralkyl oder Acyl bedeuten,
dadurch gekennzeichnet, daß man
Aminoverbindungen der allgemeinen Formel (III)

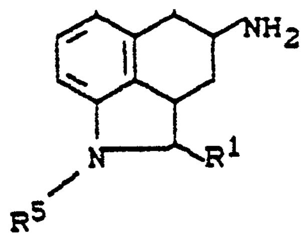

(III)

in welcher
$R^1$ die oben angegebene Bedeutung hat
und
$R^5$ - für Acyl, bevorzugt Benzoyl steht,
mit Sulfonsäurehalogeniden der allgemeinen Formel (IV)

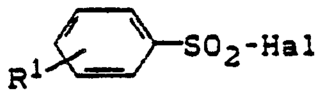

(IV)

in welcher
$R^1$ die oben angegebene Bedeutung hat
und
Hal - für Fluor, Chlor, Brom, Iod, bevorzugt für Chlor oder Brom steht,
in inerten Lösemitteln, gegebenenfalls in Anwesenheit von Basen umsetzt
und anschließend die Acylgruppe abspaltet.

9. Verfahren nach Anspruch 8, dadurch gekennezeichnet, daß man die Umsetzung im Temperaturbereich von -30° C bis +150° C durchführt.

10. Arzneimittel, enthaltend Polyhydrobenz[c,d]indol-sulfonamide der Formel

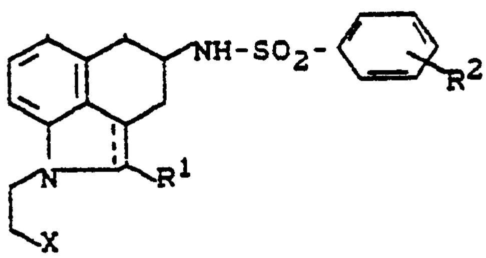

(I)

in welcher
$R^1$ - für Wasserstoff, Aryl oder Alkyl steht,
$R^2$ - für Wasserstoff, Halogen, Alkyl, Alkoxy, Trifluormethyl, Trifluormethoxy, Hydroxy, Aralkoxy oder
- für eine Gruppe der Formel

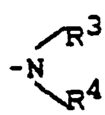

steht,
worin
$R^3$, $R^4$ - gleich oder verschieden sind und Wasserstoff, Alkyl, Aryl, Aralkyl oder Acyl bedeuten
und
X - für Cyano oder Carboxy steht,
und/oder deren Salze.

11. Arzneimittel nach Anspruch 10, enthaltend 0,5 bis 90 Gew.-% der Polyhydrobenz[c,d]indol-sulfonamide bezogen auf die Gesamtmischung.

12. Verwendung von Polyhydrobenz[c,d]indol-sulfonamide der Formel

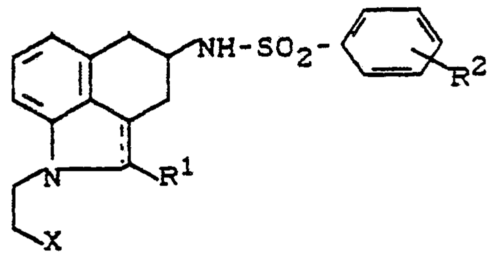

(I)

in welcher
$R^1$ - für Wasserstoff, Aryl oder Alkyl steht,
$R^2$ - für Wasserstoff, Halogen, Alkyl, Alkoxy, Trifluormethyl, Trifluormethoxy, Hydroxy, Aralkoxy oder
- für eine Gruppe der Formel

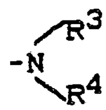

steht,
worin
$R^3$, $R^4$ - gleich oder verschieden sind und Wasserstoff, Alkyl, Aryl, Aralkyl oder Acyl bedeuten
und
X - für Cyano oder Carboxyl steht,
und/oder deren Salze,
zur Herstellung von Arzneimitteln.

13. Verwendung nach Anspruch 12 zur Herstellung von Thrombozytenaggregationshemmern.

14. Verwendung nach den Ansprüchen 12 und 13 zur Herstellung von Arzneimitteln zur Behandlung von Thrombosen, Thromboembolien, Ischämien, Asthma und Allergien.